# EUROPEAN PATENT SPECIFICATION

(11) **EP 3 799 065 B1**
(45) Date of publication and mention of the grant of the patent: **09.12.2026**
(21) Application number: 19199875.6
(22) Date of filing: 26.09.2019
(51) Int. Cl.: G16H 40/60

(54) **METHOD FOR OPERATING A MEDICAL IMAGING APPARATUS, MEDICAL IMAGING APPARATUS, COMPUTER PROGRAM AND ELECTRONICALLY READABLE STORAGE MEDIUM**
VERFAHREN ZUM BETRIEB EINER VORRICHTUNG ZUR MEDIZINISCHEN BILDGEBUNG, VORRICHTUNG ZUR MEDIZINISCHEN BILDGEBUNG, COMPUTERPROGRAMM UND ELEKTRONISCH LESBARES SPEICHERMEDIUM
PROCÉDÉ DE FONCTIONNEMENT D'UN APPAREIL D'IMAGERIE MÉDICALE, APPAREIL D'IMAGERIE MÉDICALE, PROGRAMME INFORMATIQUE ET SUPPORT D'ENREGISTREMENT LISIBLE ÉLECTRONIQUEMENT

(43) Date of publication of application: 31.03.2021
(73) Proprietor: Siemens Healthineers AG, 91301 Forchheim (DE)
(72) Inventor: Taubmann, Oliver, 91365 Weilersbach (DE); Lesyuk, Wladimir, 91054 Erlangen (DE); May, Matthias, 91056 Erlangen (DE); Uder, Michael, 91054 Erlangen (DE); Sühling, Michael, 91052 Erlangen (DE)
(74) Representative: Siemens Healthineers Patent Attorneys

(56) References cited:
- EP-A1- 2 194 486
- EP-A1- 2 374 092
- EP-A1- 3 513 738
- EP-A2- 0 726 542
- EP-B1- 2 374 092
- WO-A2-2019/157214
- GB-A- 2 586 119
- US-A1- 2018 060 535

## Description

The invention concerns a method for operating a medical imaging apparatus, comprising a control device, an input device and an output device, during an examination process of a patient. The invention further concerns a medical imaging apparatus, a computer program and an electronically readable storage medium.

In medical imaging, it is known to use a medical imaging apparatus employing at least one imaging modality to obtain image data sets of regions of interest of a patient. These image data sets are usually analysed by a radiologist, in particular some time after the image data set has been acquired. The analysis of the image data set may, for example, take place at a dedicated diagnostic work station. Later, findings or, if possible, even diagnosis, can be discussed with the patient.

The document EP2374092B1 discloses a method of identifying a region of interest from a coarse survey image.

However, many findings in image data sets acquired in examination processes of a patient cannot be fully interpreted on their own, in particular regarding chance findings. To complete the diagnostic process, additional image data sets are required, for example, using additional imaging techniques or focussing on the location of the finding. In the state of the art, the patient has to be invited for another examination process, wherein the medical imaging apparatus is manually configured to provide the additional image data required for the interpretation.

In an example, if the abdomen of an adult is imaged using computer tomography, in up to 5% of the image data sets a potential lesion of the adrenal gland is found. As such CT image data sets are usually analysed after the examination process of the patient has already been completed, a second image data set allowing for the diagnosis of the finding, for example regarding adenoma, metastasis and/or malign primary tumours, requires an additional examination process, such that a follow-up visit of the patient needs to be arranged. During this follow-up examination, for example, a so-called late washout scan requiring the administration of contrast agent may be performed.

To sum up, the diagnostic process is very slow and bothersome for the patient, in particular regarding additional medical appointments and the additional administration of contrast agent and the like.

It is an object of the present invention to improve the workflow in for examination of a patient, in particular regarding chance findings and/or other findings which are not sufficiently interpretable on their own.

This object is achieved by providing a method according to claim 1, a medical imaging apparatus according to claim 10, a computer program according to claim 11 and an electronically readable storage medium according to claim 12.

Advantageous embodiments are described in the dependent claims.

In a computer-implemented method for operating a medical imaging apparatus, comprising a control device, an input device and an output device, during an examination process of a patient, according to the invention, the method comprises the steps of
- acquiring a first image data set of a region of interest of the patient,
- in the control device, automatically evaluating the first image data set using at least one evaluation algorithm yielding evaluation information regarding at least one type of findings, the evaluation information describing the presence of at least one finding of the at least one type in the first image data set, which requires the acquisition of a second image data set for further analysis, and at least one imaging parameter for the required second image data set,
- if the evaluation information indicates a required second image data set, automatically notifying a user of the required acquisition of the second image data set using the output device, and
- after receiving, from the input device, confirmation of the acquisition of the second image data set by the user, in the same examination process, acquiring the second image data set using the at least one imaging parameter of the evaluation information.

In the method according to the invention, the first image data set of the patient is acquired as known, for example controlled by an acquisition unit of the control device. Immediately after the acquisition, in particular while the patient is still positioned in the medical imaging apparatus, the first image data set is automatically analysed in the control device using at least one evaluation algorithm, which, in essence, searches for the presence of findings of at least one type in the first image data set. The presence of these findings is an intermediate result, which, in particular, does not suffice for a diagnosis, such that additional information in form of a second image data set is required. The further information provided by the second image data set may, for example, allow for distinguishing image artefacts and/or non-malign anatomical structures from diagnostically relevant lesions. In other words, the at least one evaluation algorithm is looking for findings of a type which inspires further imaging of the finding. The findings are features, for example physical characteristics of anatomical structures and/or results of the imaging process, which require further information, in this case the second image data set, for their interpretation. The finding is a potential lesion and/or a potential image artefact.

In summary, the present invention proposes an automatic detection of findings which require further diagnostic imaging. Such findings may also be called "actionable findings". The evaluation is performed in the background after the first image data set was acquired, in particular immediately following the scan. If the result is positive, that is, a finding is present, the results are immediately output, for example at a user interface of the medical imaging apparatus. This allows for performing the acquisition of the second image data set, which may be called a work-up step, without delay. To support this workflow, at least one suitable imaging parameter, in most cases more than one imaging parameter, is also automatically determined, such that the medical imaging apparatus can be easily configured for the acquisition of the second image data set. According to the invention, the at least one imaging parameter defines a complete acquisition protocol for the medical imaging apparatus, allowing for completely automatic acquisition of the second image data set. Such acquisition protocols are already known in the state of the art and may also be employed here, in particular adapted regarding the type and/or properties of the finding and/or adapted to the current patient.

In some cases, a further acquisition may already be planned during the current examination of the patient, and it may be possible to modify this further examination to yield the second image data set. In other words, in the case that a further acquisition was already planned during this examination, the further examination may be modified depending on at least one of the at least one imaging parameter to yield the second image data set. This modification may, in some cases, also relate to the reconstruction only. In such an embodiment, no additional scan needs to be added, but an already planned further acquisition is modified to be suitable to work-up the actionable finding. However, the user is still advantageously notified regarding this change and the necessity of the second image data set. Often, however, the acquisition of the second image data set may, as described, be an additional, new scan.

It is generally noted that multiple evaluation algorithms may be used for different types of findings, while it is also possible to use one evaluation algorithm to detect the presence of multiple types of findings. For example, if the region of interest comprises several anatomical structures, findings in different anatomical structures may be differentiated and/or multiple types of findings for a certain anatomical structure may be distinguished. All those different types of findings require different additional information and thus different imaging parameters, in particular acquisition protocols, for the second image data set. Hence, imaging parameters may be allocated to certain types of findings.

Imaging parameters may, for example, describe which imaging technique to use, the field of view, contrast settings and the like. Imaging parameters may further comprise reconstruction parameters, and/or information regarding contrast agents. When the evaluation information is output to the user, if further preconditions, for example regarding the contrast agent, are required, the user is also accordingly notified.

In summary, according to the present invention, a work-up scan acquiring the second image data set can immediately be performed and its configuration is simple, fast and standardized. The risk to overlook "actionable findings" is reduced. The current invention also contributes to the establishment of an "intelligent scanner".

Additionally, time and cost are saved, since an additional summoning of the patient is unnecessary. If contrast agent has been administered to the patient regarding the acquisition of the first image data set, in some circumstances, no further contrast agent needs to be administered. For example, a so-called late washout scan may be performed using the contrast agent already administered. This leads to a further reduction of cost and time and contributes to the well-being of the patient.

These advantages are achieved by automatically detecting "actionable findings" in the medical imaging apparatus, by the immediate notification and guidance for the user and the automatical configuration of the work-up scan, that is, the acquisition of the second image data set.

While, in preferred embodiments, the medical imaging apparatus may be a computer tomography scanner, the invention may also be applied to other modalities, for example a magnetic resonance scanner, a PET imaging device, and the like. In some embodiments, the medical imaging apparatus may also be a dual-modality scanner.

The acquisition of the second image data set is proposed to the user during the examination process via the output device. The medical imaging apparatus further comprises an input device, wherein the output device and the input device may form a user interface and/or be part of a workstation of the medical imaging apparatus. The user may confirm the acquisition of the second image data set using the input device, for example by pressing a confirmation button. In the preferable case in which the imaging parameters of the evaluation information enable the fully automatic acquisition of the second image data set, the acquisition may immediately or, respecting a certain timing, in particular relating to a contrast agent, ensue.

It is, however, also possible that the at least one imaging parameter and/or additional imaging parameters are modified and/or defined depending on user input. That is, the user retains full control regarding the acquisition processes and may modify or add imaging parameters.

According to the present invention, at least one of the at least one evaluation algorithm comprises a trained function trained by a machine learning algorithm, wherein the trained function in particular comprises a deep neural network.

In general, a trained function mimics cognitive functions that humans associate with other human minds. In particular, by training based on training data, the trained function is able to adapt to new circumstances and to detect and extrapolate patterns. The trained function may, in particular, be an artificial intelligence algorithm.

In general, parameters of a trained function can be adapted by means of training. In particular, supervised training, semi-supervised training, unsupervised training, reinforcement learning and/or active learning can be used. Furthermore, representation learning (an alternative term is "feature learning") can be used. In particular, the parameters of the trained functions can be adapted iteratively by several steps of training.

In particular, the trained function can comprise a neural network, a support vector machine, a decision tree and/or a Bayesian network, and/or the trained function can be based on K-means clustering, Q-learning, genetic algorithms and/or association rules. In particular, a neural network can be a deep neural network, a convolutional neural network or a convolutional deep neural network. Furthermore, a neural network can be an adversarial network, a deep adversarial network and/or a generative adversarial network.

Regarding the trained function of the at least one evaluation algorithm, the trained function may be trained using annotated training data sets of the region of interest or subregions thereof. In many cases, the input data of the trained function may comprise image data of the first image data set. The output data may, for example, comprise modified image data, image-like data and/or other values, for example a general probability for the presence of a finding. Regarding the training of the function, input training data may comprise image data of the modality of the medical imaging apparatus, to which, for example by annotation, output training data has been associated. Input training data and output training data may form a training data set.

In preferred embodiments, the input data for the trained function may comprise image data, in particular image data of the first image data set. The output data comprises at least one probability for the presence of a finding of the at least one type. For example, when the input data comprises slice images and/or sectional images, the output data comprises corresponding probability images, in which to each data point, in particular pixel or voxel, a probability that a finding of the at least one type is present is assigned.

Generally, a method for providing the trained function may comprise:
- receiving input training data using a first training interface,
- receiving output training data using a second training interface, wherein the output training data is related to the input training data,
- training a function based on the input training data and the output training data using a training computation unit, and
- providing the trained function using a third training interface.

As already explained, the input training data may preferably comprise image data of the same modality as the medical imaging apparatus and the output training data may be annotation data associated with the image data.

Preferably, for at least one of the at least one of the at least one evaluation algorithm analysing at least one anatomical structure, image data showing the anatomical structure are extracted from the first image data set. In the following, only the extracted image data are analysed by this evaluation algorithm. For example, a landmark-based extraction may be performed. It is noted that, already in this context, artificial intelligence may be employed, for example by using a trained extraction function to extract the image data of the anatomical structure. For example, if the first image data set comprises an axial stack of two-dimensional sectional or slice images, a learning-based landmark detection may be employed to extract the relevant axial image region showing the anatomical structure. It is noted that not all of the anatomical structure has to be included in the extracted image data.

In an especially preferred embodiment, at least a part of at least one of the at least one evaluation algorithm, in particular the trained function, determines, for each data point in the analysed image data, a probability that the data point belongs to a finding of the at least one type. For example, the trained function may receive image data of the first image data set as input data, that is, image data values for certain image data points, in particular extracted pixels or voxels or even all pixels or voxels. In particular, the input data may, as discussed above, be only a part of the first image data set, for example at least one sectional or slice image. The output data of the trained function (or, generally, the part) is a probability image, which contains at least one probability value for each data point. If multiple types of findings are detected by the respective evaluation algorithm, probabilities for each type of finding may be determined separately.

In concrete embodiments, if the input data comprises sectional or slice images, each sectional or slice image may be separately input to the trained function, yielding a corresponding probability image. Regarding the use of artificial intelligence, a suitable topology for image segmentation problems may be used. To detect the presence of a finding, according to the invention, it is checked whether the maximum of the probabilities exceeds a first threshold value, such that the presence of a finding is determined when the first threshold value is exceeded by the maximum of the probabilities. In an advantageous embodiment, outlier detection may be applied to the probabilities, in particular a probability image, to exclude segmentation errors.

In particular in the case that a probability for each data point of the input image data is determined separately, such that a probability image results as output data, if the presence of a finding is determined, the position of the data point of the maximum probability can be determined as the location of the finding and/or an area surrounding the data point of the maximum probability, where the probability exceeds a second threshold, can be determined as an extension of the finding.

Generally spoken, in advantageous embodiments, at least one of the at least one evaluation algorithm additionally determines finding information describing a finding whose presence has been determined, in particular a location and/or extension and/or a characterization of the finding, wherein at least one of the at least one imaging parameter is chosen and/or adapted depending on the finding information. The determination of more information regarding the finding, apart from its presence, allows to, so to say, "tailor" the imaging parameters for the acquisition of the second image data set to the concrete finding, such that the second image data set forms an outstanding basis to later determine the nature of the finding. For example, when calculating probability images, the position where the maximum probability is found may be used as a location of the lesion, while the set of data points, in particular pixels and/or voxels, surrounding this location, for which the probability exceeds a second threshold, may quantify the extension, that is, the dimensions, of the finding as well as its posture. Such finding information may, for example, be used to determine imaging parameters describing a suitable field of view.

Regarding other finding information, which, for example, characterizes the finding, for example, a malignancy probability may be determined. In this context, it is, again, possible to employ artificial intelligence, in particular a trained characterizing function. In this case, the topology of the trained characterizing function may be chosen as one suitable for image classification problems. Training data sets may, in this case, for example comprise annotated image data of benign and malign lesions and/or image artifacts.

In the present invention, the finding information may not only be used to adapt or define certain imaging parameters, but it is also conceivable to output at least a part of the finding information to the user during notification. In this manner, the user is provided with all necessary information to decide on confirmation or non-confirmation regarding the acquisition of a second image data set.

In preferred embodiments, the image data set may be acquired using computer tomography, in particular multi-energy computer tomography. In this case, the medical imaging apparatus is a computer tomography scanner. Regarding multi-energy data, it can be advantageously used to characterize certain findings. However, the invention may also be applied to other modalities, in particular magnetic resonance imaging.

The invention further concerns a medical imaging apparatus, in particular computer tomography scanner, comprising a control device, an output device and an input device, wherein the control device is configured to perform the steps of a method according to the invention. All preferred embodiments and remarks regarding the method according to the invention correspondingly apply to the medical imaging apparatus.

In particular, the control device may comprise at least one processor and at least one storage means. In concrete embodiments, the control device may comprise an acquisition unit for controlling the acquisition of the first and second image data sets, an evaluation unit for determining the evaluation information and an input/output unit (I/O unit) for outputting the evaluation information and receiving confirmation from the user. Other functional units may be employed to realize further features of the invention.

A computer program according to the invention may be loaded into a storage means of a control device of a medical imaging apparatus and comprises instructions which, when the computer program is executed on the control device, cause the control device to carry out a method according to the invention. The computer program according to the invention may be stored on an electronically readable storage medium according to the invention, such that, when the electronically readable storage medium is used in a control device of a medical imaging apparatus, the control device carries out the steps of a method according to the invention. The electronically reada-bly storage medium may be a non-transitory medium, for example a CD ROM.

Other objects and features of the present invention will become apparent from the following detailed description considered in conjunction with the accompanying drawings. The drawings, however, are only principle sketches designed solely for the purpose of illustration and do not limit the invention. The drawings show:
- Fig. 1: a flowchart showing an embodiment of a method according to the invention,
- Fig. 2: an embodiment of an artificial neural network,
- Fig. 3: an embodiment of a convolutional neural network,
- Fig. 4: a concrete topology of a deep neural network for segmentation of findings in the adrenal gland,
- Fig. 5: a schematical drawing of a medical imaging apparatus according to the invention, and
- Fig. 6: the functional structure of a control device of the medical imaging apparatus according to fig. 5.

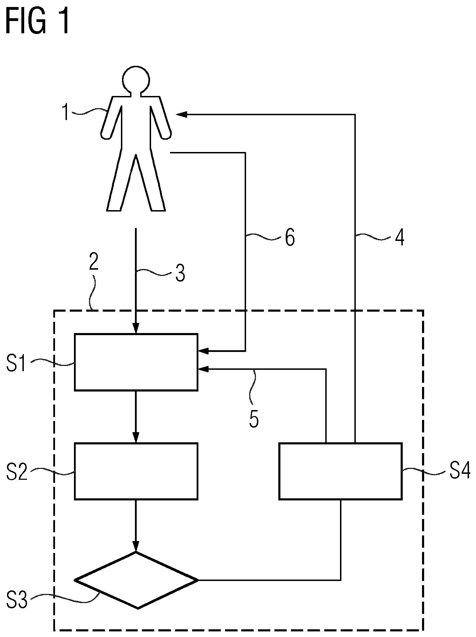

Fig. 1 shows a schematic flow chart of a method according to the invention. For better explanation, the domains of the user 1 and a medical imaging apparatus 2 (scanner) are indicated. The medical imaging apparatus 2 may, for example, be the computer tomography scanner or a magnetic resonance scanner.

The steps illustrated in fig. 1 all take place during a single examination process of a patient. That is, a patient is positioned in an imaging volume of the medical imaging apparatus 2, for example in the gantry of a computer tomography scanner or the bore of a magnetic resonance scanner. After the patient is positioned, the user 1 sets up the acquisition of a first image data set, according to arrow 3.

In a step S1, an acquisition unit of the control device of the medical imaging apparatus 2 uses the imaging parameters provided by the user 1 to acquire the first image data set of the patient. The first image data set of the patient may, for example, be a reconstructed computer tomography data set showing a region of interest of the patient, for example the abdomen, the head or the like. Once the acquisition of the first image data set is completed, including reconstruction steps, step S2 is automatically triggered, in particular while the patient still remains positioned in the medical imaging apparatus 2.

In step S2, the control device, for example an evaluation unit of the control device, evaluates the first image data set automatically in real-time to detect so-called "actionable findings". At least one evaluation algorithm is employed to detect the presence of findings which, for a complete analysis, require the acquisition of further image data of the patient, in particular a certain second image data set. In concrete embodiments, multiple types of findings may be detected, wherein each type of finding may be associated with certain imaging parameters for the corresponding second image data set. As a result of step S2, the evaluation unit outputs an evaluation information, which indicates whether a finding is present and, if a finding is present, which imaging parameters, forming a complete acquisition protocol, are expedient to acquire the corresponding second image data set. The evaluation information may further comprise finding information describing the finding, wherein such finding information is preferably also used to adapt or chose certain imaging parameters, such that, for example, a suitable contrast is given and/or the field of view for the second image data set is set correctly.

If, in step S3, it is determined that a finding requiring the acquisition of a second image data set is present, the user is notified in step S4 using the output device of a user interface, in this case a workstation, of the medical imaging apparatus 2. This notification is also indicated by arrow 4 and takes place immediately after the end of the evaluation in step S2, such that the patient is still positioned in the medical imaging apparatus 2. The notification also displays the evaluation information, in particular not only the fact that a second scan seems expedient because of a finding, but also the proposed imaging parameters as well as finding information, if it was determined as part of the evaluation information. Also, in step S4, in parallel to the notification, the medical imaging apparatus 2 is pre-configured corresponding to the imaging parameters of the evaluation information, as indicated by arrow 5.

On the output device, for example a monitor of a scanner work station, the user 1 is also asked to confirm that the second image data set of the patient is to be acquired. The user may now confirm the acquisition of the second image data set and/or may adjust imaging parameters for the acquisition of the second image data set, as indicated by arrow 6. To this end, the user 1 uses an input device of the user interface of the medical imaging apparatus **2.**

After confirmation, the acquisition unit of the control device acquires the second image data set in step S1.

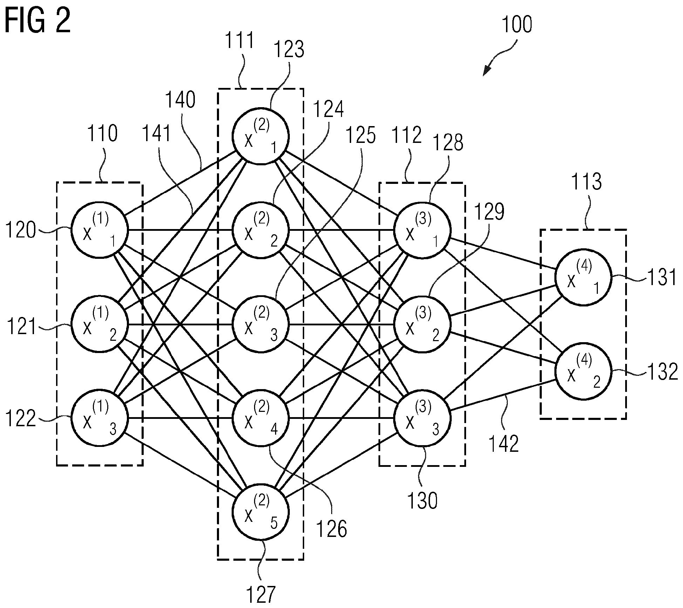

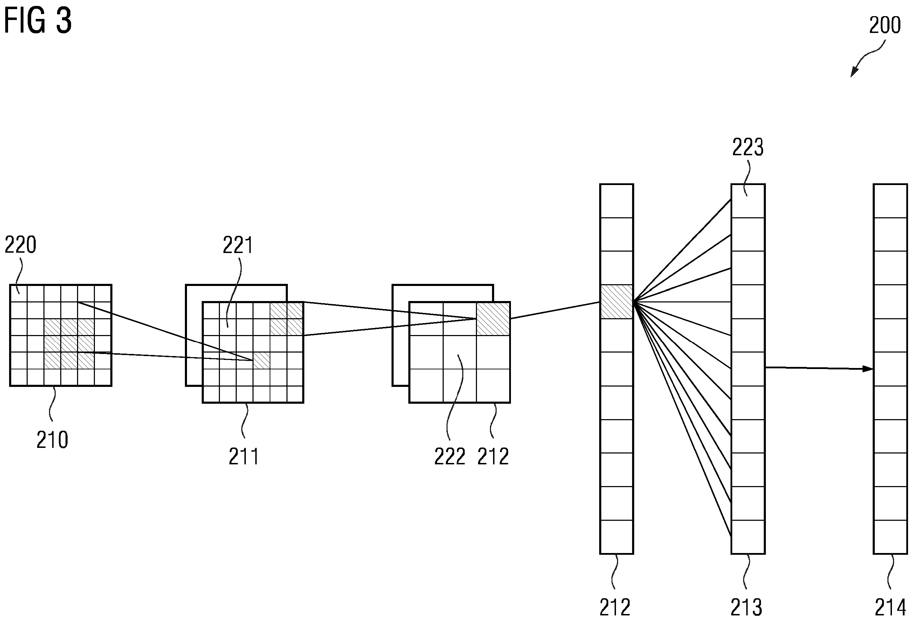

In the embodiment of fig. 1, at least one of the at least one evaluation algorithm uses artificial intelligence, in particular a trained function, which may be implemented as a neural network. Prior to explaining a concrete example, a general description of neural networks shall be given with respect to figs. 2 and **3****.**

Fig. 2 displays an embodiment of an artificial neural network 100. Alternative terms for "artificial neural network" are "neural network", "artificial neural net" or "neural net".

The artificial neural network 100 comprises nodes 120, ..., 132 and edges 140, ..., 142, wherein each edge 140, ..., 142 is a directed connection from a first node 120, ..., 132 to a second node 120, ..., 132. In general, the first node 120, ..., 132 and the second node 120, ..., 132 are different nodes 120, ..., 132, it is also possible that the first node 120, ..., 132 and the second node 120, ..., 132 are identical. For example, in Fig. 1 the edge 140 is a directed connection from the node 120 to the node 123, and the edge 142 is a directed connection from the node 130 to the node 132. An edge 140, ..., 142 from a first node 120, ..., 132 to a second node 120, ..., 132 is also denoted as "ingoing edge" for the second node 120, ..., 132 and as "outgoing edge" for the first node 120, ..., 132.

In this embodiment, the nodes 120, ..., 132 of the artificial neural network 100 can be arranged in layers 110, ..., 113, wherein the layers can comprise an intrinsic order introduced by the edges 140, ..., 142 between the nodes 120, ..., 132. In particular, edges 140, **...,** 142 can exist only between neighboring layers of nodes. In the displayed embodiment, there is an input layer 110 comprising only nodes 120, ..., 122 without an incoming edge, an output layer 113 comprising only nodes 131, 132 without outgoing edges, and hidden layers 111, 112 in-between the input layer 110 and the output layer 113. In general, the number of hidden layers 111, 112 can be chosen arbitrarily. The number of nodes 120, ..., 122 within the input layer 110 usually relates to the number of input values of the neural network, and the number of nodes 131, 132 within the output layer 113 usually relates to the number of output values of the neural network.

In particular, a (real) number can be assigned as a value to every node 120, ..., 132 of the neural network 100. Here, x⁽ⁿ⁾ᵢ denotes the value of the i-th node 120, ..., 132 of the n-th layer 110, ..., 113. The values of the nodes 120, ..., 122 of the input layer 110 are equivalent to the input values of the neural network 100, the values of the nodes 131, 132 of the output layer 113 are equivalent to the output value of the neural network 100. Furthermore, each edge 140, ..., 142 can comprise a weight being a real number, in particular, the weight is a real number within the interval [-1, 1] or within the interval [0, 1]. Here, w^{(m,n)}_{i,j} denotes the weight of the edge between the i-th node 120, ..., 132 of the m-th layer 110, ..., 113 and the j-th node 120, ..., 132 of the n-th layer 110, ..., 113. Furthermore, the abbreviation w⁽ⁿ⁾_{i,j} is defined for the weight w^{(n,n+1)}_{i,j}.

In particular, to calculate the output values of the neural network 100, the input values are propagated through the neural network. In particular, the values of the nodes 120, ..., 132 of the (n+1)-th layer 110, ..., 113 can be calculated based on the values of the nodes 120, ..., 132 of the n-th layer 110, ..., 113 by ${x}_{j}^{\left(n+1\right)} = f \left({\sum }_{i} {x}_{i}^{\left(n\right)} ⋅ {w}_{i , j}^{\left(n\right)}\right) .$

Herein, the function f is a transfer function (another term is "activation function"). Known transfer functions are step functions, sigmoid function **(e.g.** the logistic function, the generalized logistic function, the hyperbolic tangent, the Arctangent function, the error function, the smoothstep function) or rectifier functions. The transfer function is mainly used for normalization purposes.

In particular, the values are propagated layer-wise through the neural network, wherein values of the input layer 110 are given by the input of the neural network 100, wherein values of the first hidden layer 111 can be calculated based on the values of the input layer 110 of the neural network, wherein values of the second hidden layer 112 can be calculated based in the values of the first hidden layer 111, etc.

In order to set the values w^{(m,n)}_{i,j} for the edges, the neural network 100 has to be trained using training data. In particular, training data comprises training input data and training output data (denoted as tᵢ). For a training step, the neural network 100 is applied to the training input data to generate calculated output data. In particular, the training data and the calculated output data comprise a number of values, said number being equal with the number of nodes of the output layer.

In particular, a comparison between the calculated output data and the training data is used to recursively adapt the weights within the neural network 100 (backpropagation algorithm). In particular, the weights are changed according to $w {′}_{i , j}^{\left(n\right)} = {w}_{i , j}^{\left(n\right)} - γ ⋅ {δ}_{j}^{\left(n\right)} ⋅ {x}_{i}^{\left(n\right)}$ wherein y is a learning rate, and the numbers δ⁽ⁿ⁾ⱼ can be recursively calculated as ${δ}_{j}^{\left(n\right)} = \left({\sum }_{k} {δ}_{k}^{\left(n+1\right)} ⋅ {w}_{j , k}^{\left(n+1\right)}\right) ⋅ f ′ \left({\sum }_{i} {x}_{i}^{\left(n\right)} ⋅ {w}_{i , j}^{\left(n\right)}\right)$ based on δ⁽ⁿ⁺¹⁾ⱼ, if the (n+1)-th layer is not the output layer, and ${δ}_{j}^{\left(n\right)} = \left({x}_{k}^{\left(n+1\right)}-{t}_{j}^{\left(n+1\right)}\right) ⋅ f ′ \left({\sum }_{i} {x}_{i}^{\left(n\right)} ⋅ {w}_{i , j}^{\left(n\right)}\right)$ if the (n+1)-th layer is the output layer 113, wherein f' is the first derivative of the activation function, and y⁽ⁿ⁺¹⁾ⱼ is the comparison training value for the j-th node of the output layer 113.

In Fig. 3, an embodiment of a convolutional neural network 200 is shown. It is noted that the use of the term "layer" differs between classical neural networks and convolutional neural networks. For a classical neural network, the term "layer" refers only to the set of nodes forming a layer (a certain "generation" of nodes). For a convolutional neural network, the term "layer" is often used as an object that actively transforms data, in other words as a set of nodes of the same "generation" and either the set of incoming or the set of outgoing nodes.

Fig. 3 displays an embodiment of a convolutional neural network 200. In the displayed embodiment, the convolutional neural network 200 comprises an input layer 210, a convolutional layer 211, a pooling layer 212, a fully connected layer 213 and an output layer 214. Alternatively, the convolutional neural network 200 can comprise several convolutional layers 211, several pooling layers 212 and several fully connected layers 213, as well as other types of layers. The order of the layers can be chosen arbitrarily, usually fully connected layers 213 are used as the last layers before the output layer 214.

In particular, within a convolutional neural network 200 the nodes 220, ..., 224 of one layer 210, ..., 214 can be considered to be arranged as a d-dimensional matrix or as a d-dimensional image. In particular, in the two-dimensional case the value of the node 220, ..., 224 indexed with i and j in the n-th layer 210, ..., 214 can be denoted as x⁽ⁿ⁾[i,j]. However, the arrangement of the nodes 220, ..., 224 of one layer 210, ..., 214 does not have an effect on the calculations executed within the convolutional neural network 200 as such, since these are given solely by the structure and the weights of the edges.

In particular, a convolutional layer 211 is characterized by the structure and the weights of the incoming edges forming a convolution operation based on a certain number of kernels. In particular, the structure and the weights of the incoming edges are chosen such that the values x⁽ⁿ⁾ₖ of the nodes 221 of the convolutional layer 211 are calculated as a convolution x⁽ⁿ⁾ₖ = Kₖ * x⁽ⁿ⁻¹⁾ based on the values x⁽ⁿ⁻¹⁾ of the nodes 220 of the preceding layer 210, where the convolution * is defined in the two-dimensional case as ${x}_{k}^{\left(n\right)} \left[i, j\right] = \left({K}_{k}*{x}^{\left(n-1\right)}\right) \left[i, j\right] = {\sum }_{i ′} {\sum }_{j ′} {K}_{k} \left[i′,j′\right] ⋅ {x}^{\left(n-1\right)} \left[i-i′, j-j′\right] .$

Here the k-th kernel Kₖ is a d-dimensional matrix (in this embodiment a two-dimensional matrix), which is usually small compared to the number of nodes 220, ..., 224 (e.g. a 3x3 matrix, or a 5x5 matrix). In particular, this implies that the weights of the incoming edges are not independent, but chosen such that they produce said convolution equation. In particular, for a kernel being a 3x3 matrix, there are only 9 independent weights (each entry of the kernel matrix corresponding to one independent weight), irrespectively of the number of nodes 220, ..., 224 in the respective layer 210, ..., 214. In particular, for a convolutional layer 211 the number of nodes 221 in the convolutional layer is equivalent to the number of nodes 220 in the preceding layer 210 multiplied with the number of kernels.

If the nodes 220 of the preceding layer 210 are arranged as a d-dimensional matrix, using a plurality of kernels can be interpreted as adding a further dimension (denoted as "depth" dimension), so that the nodes 221 of the convolutional layer 221 are arranged as a (d+1)-dimensional matrix. If the nodes 220 of the preceding layer 210 are already arranged as a (d+1)-dimensional matrix comprising a depth dimension, using a plurality of kernels can be interpreted as expanding along the depth dimension, so that the nodes 221 of the convolutional layer 221 are arranged also as a (d+1)-dimensional matrix, wherein the size of the (d+1)-dimensional matrix with respect to the depth dimension is by a factor of the number of kernels larger than in the preceding layer 210.

The advantage of using convolutional layers 211 is that spatially local correlation of the input data can exploited by enforcing a local connectivity pattern between nodes of adjacent layers, in particular by each node being connected to only a small region of the nodes of the preceding layer.

In the displayed embodiment, the input layer 210 comprises 36 nodes 220, arranged as a two-dimensional 6x6 matrix. The convolutional layer 211 comprises 72 nodes 221, arranged as two two-dimensional 6x6 matrices, each of the two matrices being the result of a convolution of the values of the input layer with a kernel. Equivalently, the nodes 221 of the convolutional layer 211 can be interpreted as arranges as a three-dimensional 6x6x2 matrix, wherein the last dimension is the depth dimension.

A pooling layer 212 can be characterized by the structure and the weights of the incoming edges and the activation function of its nodes 222 forming a pooling operation based on a nonlinear pooling function f. For example, in the two dimensional case the values x⁽ⁿ⁾ of the nodes 222 of the pooling layer 212 can be calculated based on the values x⁽ⁿ⁻¹⁾ of the nodes 221 of the preceding layer 211 as ${x}^{\left(n\right)} \left[i, j\right] = f \left({x}^{\left(n-1\right)}\left[{id}_{1}, {jd}_{2}\right], …, {x}^{\left(n-1\right)}\left[{id}_{1}+{d}_{1}-1, {jd}_{2}+{d}_{2}-1\right]\right)$

In other words, by using a pooling layer 212 the number of nodes 221, 222 can be reduced, by replacing a number d₁·d₂ of neighboring nodes 221 in the preceding layer 211 with a single node 222 being calculated as a function of the values of said number of neighboring nodes in the pooling layer. In particular, the pooling function f can be the max-function, the average or the L2-Norm. In particular, for a pooling layer 212 the weights of the incoming edges are fixed and are not modified by training.

The advantage of using a pooling layer 212 is that the number of nodes 221, 222 and the number of parameters is reduced. This leads to the amount of computation in the network being reduced and to a control of overfitting.

In the displayed embodiment, the pooling layer 212 is a max-pooling, replacing four neighboring nodes with only one node, the value being the maximum of the values of the four neighboring nodes. The max-pooling is applied to each d-dimensional matrix of the previous layer; in this embodiment, the max-pooling is applied to each of the two two-dimensional matrices, reducing the number of nodes from 72 to 18.

A fully-connected layer 213 can be characterized by the fact that a majority, in particular, all edges between nodes 222 of the previous layer 212 and the nodes 223 of the fully-connected layer 213 are present, and wherein the weight of each of the edges can be adjusted individually.

In this embodiment, the nodes 222 of the preceding layer 212 of the fully-connected layer 213 are displayed both as two-dimensional matrices, and additionally as non-related nodes (indicated as a line of nodes, wherein the number of nodes was reduced for a better presentability). In this embodiment, the number of nodes 223 in the fully connected layer 213 is equal to the number of nodes 222 in the preceding layer 212. Alternatively, the number of nodes 222, 223 can differ.

Furthermore, in this embodiment the values of the nodes 224 of the output layer 214 are determined by applying the Softmax function onto the values of the nodes 223 of the preceding layer 213. By applying the Softmax function, the sum of the values of all nodes 224 of the output layer is 1, and all values of all nodes 224 of the output layer are real numbers between 0 and 1. In particular, if using the convolutional neural network 200 for categorizing input data, the values of the output layer can be interpreted as the probability of the input data falling into one of the different categories.

A convolutional neural network 200 can also comprise a ReLU (acronym for "rectified linear units") layer. In particular, the number of nodes and the structure of the nodes contained in a ReLU layer is equivalent to the number of nodes and the structure of the nodes contained in the preceding layer. In particular, the value of each node in the ReLU layer is calculated by applying a rectifying function to the value of the corresponding node of the preceding layer. Examples for rectifying functions are f(x) = max(0,x), the tangent hyperbol-ics function or the sigmoid function.

In particular, convolutional neural networks 200 can be trained based on the backpropagation algorithm. For preventing overfitting, methods of regularization can be used, e.g. dropout of nodes 220, ..., 224, stochastic pooling, use of artificial data, weight decay based on the L1 or the L2 norm, or max norm constraints.

While the current invention may be applied to different types of findings and different regions of interest, in the following, a learning-based evaluation algorithm, in particular comprising one deep neural network as a trained function, which detects findings in the adrenal glands in a first image data set of the abdomen of a patient, shall be presented. The evaluation algorithm is configured to detect, quantify and potentially characterize a finding, for example a potential lesion, a potential artefact and/or potential properties of an adrenal gland, for example potentially hypertrophic adrenal glands. The evaluation algorithm is applied by the control device automatically after each thorax and/or abdomen scan of the patient. If the result is positive, that is, the evaluation information indicates the presence of a finding, the user is notified on the user interface and it is proposed to perform a late wash out scan for acquisition of the second image data set, which is also (in step S4 of fig. 1) already pre-configured.

In a first step of the evaluation algorithm for the adrenal glands, the relevant image data is extracted from the first image data set, i.e. image data showing the adrenal gland as anatomic structure. As an input for this step, the first image data set, in this concrete example a reconstructed computer tomography data set of the thorax/abdomen area, is used. The first image data set is provided as an axial stack of sectional images. Using a landmark detection, which may be based on machine learning, the relevant axial image part, that is, a subset of the sectional images located between the uppermost position of the liver and the middle of the lower-positioned kidney, is determined.

In a second step, each of the extracted two-dimensional sectional images are used as input data for a trained function, in this case a deep neural network, whose output data is a sectional image of the same size, in this case a probability image. In the probability image, each pixel (data point) is assigned a predicted probability that this pixel is part of a finding.

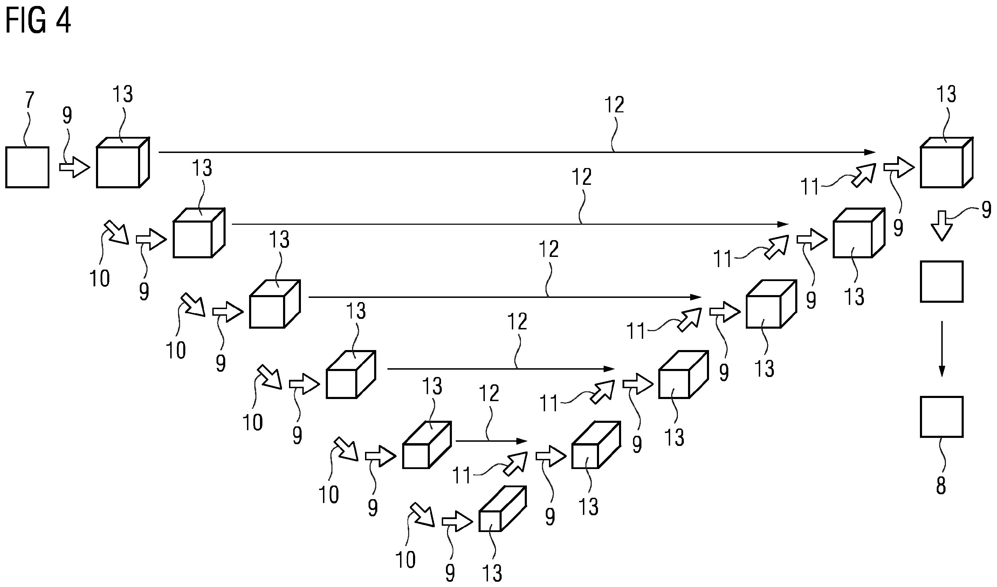

Fig. 4 shows an exemplary topology of a deep neural network which can be used to determine probability images in this second step of the evaluation algorithm. The input data 7 are, as explained, axial sectional images of the first image data set, while the output data 8 are probability images. The arrows 9 each describe a layer performing the operation [convolution (3x3) + batch normalization + leaky ReLU]², the arrows 10 symbolize a max-pooling layer (2x2), the arrows 11 indicate the operation of up-sampling (2x2) + convolution (3x3, depth/2) and the arrows 12 stand for skip connections. The respective boxes 13 indicate the evolution of the number of nodes, see as well the explanation with regard to fig. 3.

Of course, also different topologies which are used for image segmentation problems may be employed. Regarding the training of the trained function, segmentation masks of adrenal gland findings, which have been generated by manual contouring, can be used.

In a third step of the evaluation algorithm, the probability images are interpreted to determine the presence of a finding and, if a finding is present, corresponding finding information. In this embodiment, the maximum of all probabilities in the probability images is compared to a first threshold. If the maximum probability exceeds this first threshold, the detection is determined as positive. Additionally, the location, where the maximum probability has been determined, is used as a location of the finding, and the sub-set of the pixels around this location whose predicted probabilities exceed a second threshold, is interpreted as defining the extensions of the finding, in particular its dimensions.

In an optional embodiment, if the first image data set has been acquired using dual-energy computer tomography, the characterisation of the finding can also be implemented. The spectral information in the area of the finding can be used as input data for a further trained function, in particular a further neural network, which determines, as output data, a malignancy probability for the finding, in particular the potential lesion. For such a neural network, any topology which is known for image classification problems may be used. The neural network can be trained on the basis of annotated benign and malign adrenal gland lesions and/or image artefacts.

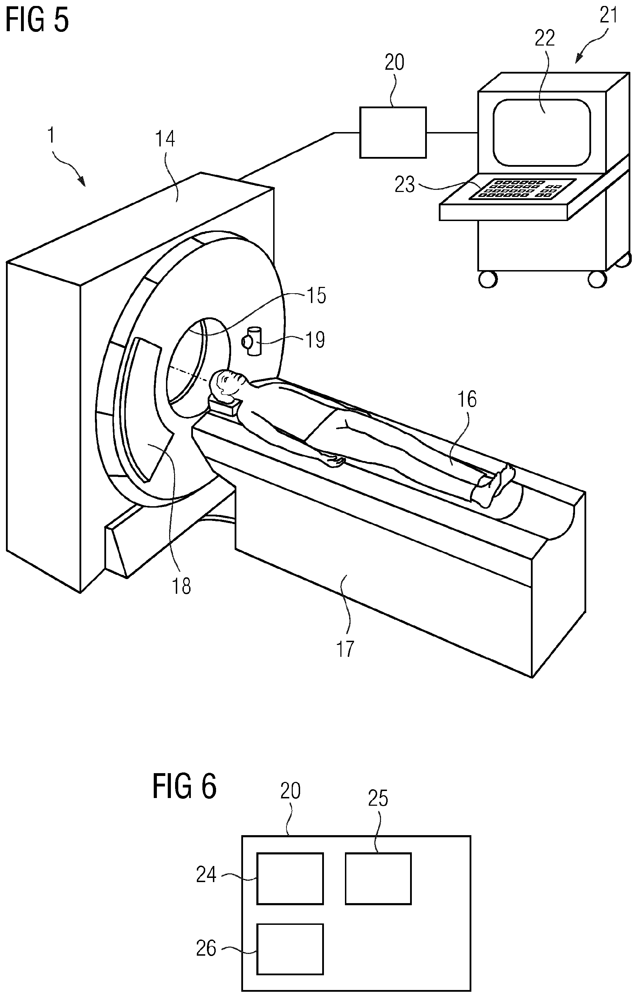

Fig. 5 shows an embodiment of a medical imaging apparatus 1 according to the present invention, in this case a computer tomography scanner. The medical imaging apparatus thus comprises a gantry 14 having an opening 15 for a patient 16 which may be positioned on a respective patient table 17. In the gantry 14, an x-ray detector 18 and an x-ray source 19 are rotatably mounted to scan the region of interest of the patient 16 positioned in the opening 15.

The operation of the medical imaging apparatus 1 is controlled by a control device 20, which is configured to perform a method according to the invention. The control device is also connected to a user interface 21 of the medical imaging apparatus 1, which in turn comprises an output device 22 and an input device 23. The user interface 21 may, for example, be a scanner work station.

The user interface 21 as well as other components of the medical imaging apparatus 1 may be connected to the control device via corresponding interfaces.

Fig. 6 shows the functional structure of the control device 20. In particular, the control device 20 comprises, as in principle known, an acquisition unit 24 controlling the acquisition of image data sets according to imaging parameters. The acquisition unit 24 may also include a reconstruction sub unit for reconstructing three-dimensional computer tomography data sets. The acquisition unit 24 is adapted to perform the step S1 in fig. 1.

An evaluation unit 25 is provided to perform the step S2 according to fig. 1. Triggered by the completion of the acquisition of a first image data set, the evaluation unit 25 applies the at least one evaluation algorithm to the first image data set to derive the evaluation information.

The input/output unit 26, among other tasks, is configured to notify the user 1 via the output device 22 if a finding is detected (steps S3 and S4 in fig. 1) and to receive corresponding input from the input device 23, in particular a confirmation.

Although the present invention has been described in detail with reference to the preferred embodiment, the present invention is not limited by the disclosed examples from which the skilled person is able to derive other variations without departing from the scope of the invention as defined by the patent claims.

## Claims

1. Method for operating a computed tomography medical imaging apparatus (2), comprising a control device (20), an input device (23) and an output device (22), during an examination process of a patient (16), comprising the steps of
- acquiring a first image data set of a region of interest of the patient (16),
- in the control device (20), automatically evaluating the first image data set using at least one evaluation algorithm yielding evaluation information regarding at least one type of findings, the finding being a potential lesion and/or a potential image artifact and the evaluation information describing the presence of at least one finding of the type in the first image data set, which requires the acquisition of a second image data set for further analysis, and at least one imaging parameter for the required second image data set, wherein the first image data set comprises at least one sectional or slice image and wherein the presence of the finding is an intermediate result, which does not suffice for a diagnosis, such that additional information in form of a second image data set is required,
- if the evaluation information indicates a required second image data set, automatically notifying a user (1) of the required acquisition of the second image data set using the output device (22),
- after confirmation of the acquisition of the second image data set by the user (1), in the same examination process, acquiring the second image data set using the at least one imaging parameter of the evaluation information,
wherein the at least one imaging parameter defines a complete acquisition protocol for the medical imaging apparatus (2),
allowing completely automatic acquisition of the second image data set and wherein different types of findings require different acquisition protocols for the second image data set,
**characterized in that**
at least one of the at least one evaluation algorithm comprises a trained function trained by a machine learning algorithm, wherein the output data of the trained function comprises corresponding probability images in which to each data point, namely pixel or voxel, a probability that a finding of the at least one type is present is assigned, wherein, if the maximum of the probabilities exceeds a first threshold value, the presence of a finding is determined when a first threshold value is exceeded by the maximum of the probabilities.

2. Method according to claim 1, **characterized in that** the at least one imaging parameter and/or additional imaging parameters are modified and/or defined depending on user (1) input.

3. Method according to one of the preceding claims, **characterized in that** the trained function comprises a deep neural network.

4. Method according to any of the preceding claims, **characterized in that** the trained function is trained using annotated training data sets of the region of interest or subregions thereof.

5. Method according to one of the preceding claims, **characterized in that**, for at least one of the at least one evaluation algorithm analyzing at least one anatomical structure, image data showing the anatomical structure are extracted from the first image data set.

6. Method according to one of the preceding claims, **characterized in that**, if the presence of a finding is determined, the position of the data point of the maximum probability is determined as the location of the finding and/or an area surrounding the data point of the maximum probability, where the probability exceeds a second threshold, is determined as an extension of the finding.

7. Method according to one of the preceding claims, **characterized in that** at least one of the at least one evaluation algorithm additionally determines finding information describing a finding whose presence has been determined.

8. Method according to claim 7, **characterized in that** at least a part of the finding information is also output to the user (1).

9. Method according to one of the preceding claims, **characterized in that** the image data sets are acquired using multi-energy computed tomography.

10. A computed tomography scanner, comprising a control device (20), an input device (23) and an output device (22), wherein the control device (20) is configured to perform the steps of a method according to one of the preceding claims.

11. Computer program, comprising instructions which, when the computer program is executed on a control device (20) of a medical imaging apparatus (2), cause the control device (20) to carry out a method according to any of the claims 1 to 9.

12. Electronically readable storage medium, in which a computer program according to claim 11 is stored.

## Patentansprüche

1. Verfahren zum Betreiben einer medizinischen Computertomographie-Bildgebungseinrichtung (2), die eine Steuervorrichtung (20), eine Eingabevorrichtung (23) und eine Ausgabevorrichtung (22) umfasst, während eines Untersuchungsprozesses eines Patienten (16), umfassend die folgenden Schritte:
- Erfassen eines ersten Bilddatensatzes einer Interessensregion des Patienten (16),
- in der Steuereinrichtung (20), automatisches Auswerten des ersten Bilddatensatzes unter Verwendung mindestens eines Auswertungsalgorithmus, der Auswertungsinformationen bezüglich mindestens einer Art von Befunden liefert, wobei es sich bei dem Befund um eine potenzielle Läsion und/oder ein potentielles Bildartefakt handelt, und die Auswertungsinformationen das Vorliegen mindestens eines Befundes der Art in dem ersten Bilddatensatz beschreiben, was die Erfassung eines zweiten Bilddatensatzes zur weiteren Analyse erfordert; und mindestens einen Bildgebungsparameter für den erforderlichen zweiten Bilddatensatz, wobei der erste Bilddatensatz mindestens ein Schnitt- oder Schichtbild umfasst, und wobei das Vorliegen des Befundes ein Zwischenergebnis ist, das für eine Diagnose nicht ausreicht, so dass zusätzliche Informationen in Form eines zweiten Bilddatensatzes erforderlich sind,
- falls die Auswertungsinformationen einen erforderlichen zweiten Bilddatensatz angeben, automatisches Benachrichtigen eines Benutzers (1) über die erforderliche Erfassung des zweiten Bilddatensatzes unter Verwendung der Ausgabevorrichtung (22);
- nach Bestätigung der Erfassung des zweiten Bilddatensatzes durch den Benutzer (1), in demselben Untersuchungsprozess, Erfassen des zweiten Bilddatensatzes unter Verwendung des mindestens einen Bildgebungsparameters der Auswertungsinformationen,
wobei der mindestens eine Bildgebungsparameter ein vollständiges Erfassungsprotokoll für die medizinische Bildgebungseinrichtung (2) definiert, das eine vollständig automatische Erfassung des zweiten Bilddatensatzes ermöglicht, und wobei unterschiedliche Arten von Befunden unterschiedliche Erfassungsprotokolle für den zweiten Bilddatensatz erfordern,
**dadurch gekennzeichnet, dass**
mindestens einer des mindestens einen Auswertungsalgorithmus eine trainierte Funktion umfasst, die durch einen Maschinenlernalgorithmus trainiert wird, wobei die Ausgabedaten der trainierten Funktion entsprechende Wahrscheinlichkeitsbilder umfassen, in denen jedem Datenpunkt, nämlich Pixel oder Voxel, eine Wahrscheinlichkeit zugewiesen wird, dass ein Befund der mindestens einen Art vorliegt,
wobei, falls das Maximum der Wahrscheinlichkeiten einen ersten Schwellenwert überschreitet, das Vorhandensein eines Befundes bestimmt wird, wenn ein erster Schwellenwert durch das Maximum der Wahrscheinlichkeiten überschritten wird.

2. Verfahren nach Anspruch 1, **dadurch gekennzeichnet, dass** der mindestens eine Bildgebungsparameter und/oder zusätzliche Bildgebungsparameter in Abhängigkeit von der Eingabe durch den Benutzer (1) modifiziert und/oder definiert werden.

3. Verfahren nach einem der vorhergehenden Ansprüche, **dadurch gekennzeichnet, dass** die trainierte Funktion ein tiefes neuronales Netzwerk umfasst.

4. Verfahren nach einem der vorhergehenden Ansprüche, **dadurch gekennzeichnet, dass** die trainierte Funktion unter Verwendung von annotierten Trainingsdatensätzen der Interessensregion oder von Unterregionen davon trainiert wird.

5. Verfahren nach einem der vorhergehenden Ansprüche, **dadurch gekennzeichnet, dass** für mindestens einen des mindestens einen Auswertungsalgorithmus, der mindestens eine anatomische Struktur analysiert, Bilddaten, die die anatomische Struktur zeigen, aus dem ersten Bilddatensatz extrahiert werden.

6. Verfahren nach einem der vorhergehenden Ansprüche, **dadurch gekennzeichnet, dass**, falls das Vorhandensein eines Befundes bestimmt wird, die Position des Datenpunkts der maximalen Wahrscheinlichkeit als der Ort des Befundes bestimmt wird und/oder ein den Datenpunkt der maximalen Wahrscheinlichkeit umgebender Bereich, bei dem die Wahrscheinlichkeit eine zweite Schwelle überschreitet, als Erweiterung des Befundes bestimmt wird.

7. Verfahren nach einem der vorhergehenden Ansprüche, **dadurch gekennzeichnet, dass** mindestens einer des mindestens einen Auswertungsalgorithmus zusätzlich eine Befundinformation bestimmt, die einen Befund beschreiben, dessen Vorhandensein bestimmt wurde.

8. Verfahren nach Anspruch 7, **dadurch gekennzeichnet, dass** zumindest ein Teil der Befundinformation auch an den Benutzer (1) ausgegeben wird.

9. Verfahren nach einem der vorhergehenden Ansprüche, **dadurch gekennzeichnet, dass** die Bilddatensätze unter Verwendung einer Multi-Energie-Computertomographie erfasst werden.

10. Computertomographiescanner, umfassend eine Steuervorrichtung (20), eine Eingabevorrichtung (23) und eine Ausgabevorrichtung (22), wobei die Steuervorrichtung (20) dazu ausgelegt ist, die Schritte eines Verfahrens nach einem der vorhergehenden Ansprüche durchzuführen.

11. Computerprogramm, umfassend Anweisungen, die bei Ausführung des Computerprogramms auf einer Steuervorrichtung (20) einer medizinischen Bildgebungseinrichtung (2) bewirken, dass die Steuervorrichtung (20) ein Verfahren nach einem der Ansprüche 1 bis 9 ausführt.

12. Elektronisch lesbares Speichermedium, in dem ein Computerprogramm nach Anspruch 11 gespeichert ist.

## Revendications

1. Procédé pour faire fonctionner une installation (2) d'imagerie médicale assistée par ordinateur de tomodensitométrie, comprenant un dispositif (20) de commande, un dispositif (23) d'entrée et un dispositif (22) de sortie, pendant une opération d'examen d'un patient (16), comprenant les stades de
- acquérir un premier ensemble de données d'image d'une région du patient (16) à laquelle on s'intéresse,
- dans le dispositif (20) de commande, évaluer automatiquement le premier ensemble de données d'image en utilisant au moins un algorithme d'évaluation fournissant une information d'évaluation concernant au moins un type de constatations, la constatation étant une lésion potentielle et/ou un artéfact d'image potentiel et l'information d'évaluation décrivant la présence d'au moins une constatation du type dans le premier ensemble de données d'image, qui requiert l'acquisition d'un deuxième ensemble de données d'image pour autre analyse et au moins un paramètre d'imagerie pour le deuxième ensemble de données d'image requis, dans lequel le premier ensemble de données d'image comprend au moins une image sectionnelle ou de tranche et dans lequel la présence de la constatation est un résultat intermédiaire, qui ne suffit pas pour un diagnostic, de sorte que de l'information supplémentaire sous la forme d'un deuxième ensemble de données d'image est requis,
- si l'information d'évaluation indique un deuxième ensemble de données d'image requis, notifier automatiquement à un utilisateur (1) l'acquisition requise du deuxième ensemble de données d'image en utilisant le dispositif (22) de sortie,
- après confirmation de l'acquisition du deuxième ensemble de données d'image par l'utilisateur (1), dans la même opération .
d'examen, acquérir le deuxième ensemble de données d'image en utilisant le au moins un paramètre d'imagerie de l'information d'évaluation,
dans lequel le au moins un paramètre d'imagerie définit un protocole complet d'acquisition pour l'installation (2) d'imagerie médicale permettant une acquisition automatique complètement du deuxième ensemble de donnés d'image et dans lequel des types différents de constatations requièrent des protocoles différents d'acquisition pour le deuxième ensemble de données d'image,
**caractérisé en ce qu'**
au moins l'un du au moins un algorithme d'évaluation comprend une fonction entraînée, entraînée par un algorithme d'apprentissage automatique, dans lequel la donnée de sortie de la fonction entraînée comprend des images correspondantes de probabilité, dans lesquelles à chaque point de données, à savoir pixel ou voxel, est assignée une probabilité, qu'une constatation du au moins un type est présente, dans lequel, si le maximum des probabilités dépasse une première valeur de seuil, la présence d'une constatation est déterminée lorsqu'une première valeur de seuil est dépassée par le maximum des probabilités.

2. Procédé suivant la revendication 1, **caractérisé en ce que** le au moins un paramètre d'imagerie et/ou des paramètres supplémentaires d'imagerie sont modifiés et/ou définis en fonction de l'entrée d'utilisateur (1).

3. Procédé suivant l'une des revendications précédentes, **caractérisé en ce que** la fonction entraînée comprend un réseau neuronal profond.

4. Procédé suivant l'une des revendications précédentes, **caractérisé en ce que** la fonction entraînée est entraînée en utilisant des ensembles de données d'entraînement annotés de la région à laquelle on s'intéresse ou de ses sous-régions. .

5. Procédé suivant l'une des revendications précédentes, **caractérisé en ce que**, pour au moins l'un du au moins un algorithme d'évaluation analysant au moins une structure anatomique, des données d'image représentant la structure anatomique sont extraites du premier ensemble de données d'image.

6. Procédé suivant l'une des revendications précédentes, **caractérisé en ce que**, si la présence d'une constatation est déterminée, la position du point de données de la probabilité maximum est déterminée comme l'emplacement de la constatation et/ou une surface entourant le point de données de la probabilité maximum, où la probabilité dépasse un deuxième seuil, est déterminée comme une extension de la constatation.

7. Procédé suivant l'une des revendications précédentes, **caractérisé en ce qu'**au moins l'un du au moins un algorithme d'évaluation détermine supplémentairement une information de constatation décrivant une constatation, dont la présence a été déterminée.

8. Procédé suivant la revendication 7, **caractérisé en ce qu'**au moins une partie de l'information de constatation est donnée également à l'utilisateur (1).

9. Procédé suivant l'une des revendications précédentes, **caractérisé en ce que** les ensembles de données d'image sont acquis en utilisant une tomodensitométrie assistée par ordinateur à multiples énergies.

10. Un scanner de tomodensitométrie assisté par ordinateur, comprenant un dispositif (20) de commande, un dispositif (23) d'entrée et un dispositif (22) de sortie, dans lequel le dispositif . (20) de commande est configuré pour effectuer les stades d'un procédé suivant l'une des revendications précédentes.

11. Programme d'ordinateur, comprenant des instructions, qui, lorsque le programme d'ordinateur est exécuté sur un dispositif (20) de commande d'une installation (2) d'imagerie médicale, font que le dispositif (20) de commande exécute un procédé suivant l'une quelconque des revendications 1 à 9.

12. Support de mémoire déchiffrable électroniquement, dans lequel un programme d'ordinateur suivant la revendication 11 est mis en mémoire. .
